# EUROPÄISCHE PATENTSCHRIFT

(11) **EP 3 792 676 B1**
(45) Veröffentlichungstag und Bekanntmachung des Hinweises auf die Patenterteilung: **19.07.2023**
(21) Anmeldenummer: 20192333.1
(22) Anmeldetag: 24.08.2020
(51) Int. Cl.: G02B 23/24, G02B 5/20

(54) **ENDOSKOP MIT OPTISCHER FILTERANORDNUNG UND VERWENDUNG**
ENDOSCOPE WITH OPTICAL FILTER ARRANGEMENT AND USE
ENDOSCOPE POURVU D'AGENCEMENT FILTRANT OPTIQUE ET UTILISATION

(30) Priorität: 28.08.2019 DE 102019123053
(43) Veröffentlichungstag der Anmeldung: 17.03.2021
(73) Patentinhaber: OLYMPUS Winter & Ibe GmbH, 22045 Hamburg (DE)
(72) Erfinder: ZHAO, Jianxin, 22399 Hamburg (DE)
(74) Vertreter: Seemann & Partner Patentanwälte mbB

(56) Entgegenhaltungen:
- DE-A1-102009 011 681
- DE-A1-102015 003 681
- US-A1- 2002 049 366

## Beschreibung

Die Erfindung betrifft ein Endoskop mit einer optischen Filteranordnung sowie eine Verwendung des Endoskops.

Endoskope werden zur optischen Untersuchung, unter anderem im medizinischen Bereich, verwendet, wobei in vielen Fällen optische Filter verwendet werden, um spezielle Untersuchungsmodalitäten zu realisieren. Zu den optischen Filtern gehören Farbfilter ebenso wie Bandpass- oder Kantenfilter, Polarisationsfilter sowie UV- und Infrarot-Filter (IR-Filter). Letztere ermöglichen es, die Tatsache, dass Endoskope auch im Infrarotbereich eingesetzt werden können, auszunutzen, beispielsweise für die Anwendung der Fluoreszenzangiographie mittels Indocyaningrün, dessen Fluoreszenzspektrum im nah-infraroten Bereich liegt. Bei den IR-Laparoskopen der Anmelderin wird ein IR-Filter zwischen der Feldmaske und dem Okular positioniert.

Für verschiedene Anwendungen müssen Ärzte daher zwei unterschiedliche Laparoskope für die normale Laparoskopie bei sichtbarem Licht und für die Infrarot-Laparoskopie besitzen und zwischen diesen wechseln. Es wäre daher wünschenswert, beide Anwendungen mit einem Endoskop ausführen zu können.

Aus US 2012/0257030 A1 ist eine Endoskopvorrichtung bekannt, bei der Licht aus einer Lichtquelle abwechselnd durch verschiedene, in einem Filterrad angeordnete, Farbfilter durchgeleitet wird, so dass das Illuminationslicht bereits die entsprechende Farbcharakteristik aufweist. Da das Filterrad größer ist als die weiße Lichtquelle, ist diese Vorrichtung recht großbauend. Aus US 4,878,113 A ist eine Endoskopvorrichtung bekannt, die ebenfalls bei der Lichtquelle ein Filterrad mit verschiedenfarbigen Filtern im sichtbaren Bereich aufweist.

Aus DE 10 2015 003 681 A1 ist eine Vorrichtung zum Aufnehmen eines Bildes eines Objektfelds an einem menschlichen oder tierischen Körper bekannt, welche ein schwenkbares Filter aufweist, welches durch Schwenkung um eine Schwenkachse, die senkrecht zur Längsachse des Strahlengangs der Vorrichtung ausgerichtet ist, in den Strahlengang hinein und aus dem Strahlengang wieder herausgeschwenkt werden kann. So erfolgt in einem Schwenkzustand eine Filterung, im anderen Schwenkzustand erfolgt keine Filterung.

DE 10 2009 011 681 A1 offenbart einen Wechsler für optische Elemente mit einer Rotationsachse zum Einsatz in einem Operationsmikroskop, einem Stereomikroskop, einem Mikroskop, einem Endoskop oder einem Stereoendoskop. Der Wechsler bringt ein optisches Element durch eine oder unter Beteiligung einer Rotationsbewegung in einen optischen Strahlengang ein.

In US 2002/049366 A1 wird eine Vorrichtung zum Positionieren wenigstens einer optischen Komponente innerhalb eines endoskopischen Systems beschrieben, das ein Gehäuse aufweist, durch welches eine optische Achse verläuft und in dem die wenigstens eine optische Komponente angeordnet ist. Die optische Komponente kann um eine Schwenkachse in einen Strahlengang des endoskopischen Systems eingeschwenkt und wieder herausgeschwenkt werden. Die Schwenkachse verläuft schräg zur optischen Achse.

Die der vorliegenden Erfindung zugrundeliegende Aufgabe liegt gegenüber diesem Stand der Technik darin, in einem einzigen Endoskop verschiedene Filtereigenschaften zu realisieren, ohne die Baugröße des Endoskops vergrößern zu müssen.

Diese Aufgabe wird durch ein Endoskop mit einer optischen Filteranordnung gelöst, welche in einer Halterung mehrere optische Filter mit unterschiedlichen Filtereigenschaften umfasst, und in wenigstens einem Strahlengang des Endoskops in Lichteinfallsrichtung in oder hinter einem Objektiv des Endoskops und vor einer Bildaufnahmeeinheit des Endoskops oder einem Okular des Endoskops um eine Drehachse drehbar angeordnet ist, wobei die Filteranordnung ausgebildet ist, bei Drehung der Filteranordnung um 90° um die Drehachse von einer ersten Solldrehposition in eine zweite Solldrehposition einen Wechsel von einer ersten Filtereigenschaft zu einer zweiten Filtereigenschaft zu vollziehen, wobei die Filteranordnung zwei, drei oder vier plane optische Filter aufweist, die von der Halterung gehalten werden und um 90° zueinander gedreht angeordnet sind, wobei in der ersten Solldrehposition wenigstens eines der planen optischen Filter mit der ersten Filtereigenschaft und in der zweiten Solldrehposition wenigstens ein anderes der planen optischen Filter mit der zweiten Filtereigenschaft im Strahlengang steht, wobei die Drehachse der Filteranordnung senkrecht zu dem wenigstens einen Strahlengang des Endoskops ausgerichtet ist.

Unter einer Filtereigenschaft wird im Rahmen der vorliegenden Erfindung die spektrale oder polare Filtercharakteristik des optischen Filters verstanden, also u.a., in welchem Wellenlängenbereich das Filter durchlässig ist. Dies kann vom Infrarotbereich bis in den Ultraviolettbereich gehen und gegebenenfalls Polarisationsfilterung, bspw. für lineare oder zirkulare Polarisation, einschließen. Die ersten und zweiten Filtereigenschaften müssen sich allerdings unterscheiden.

Die Erfindung beruht auf dem Grundgedanken, dass eine drehbare Filteranordnung durch eine einfache Drehung derselben um 90° zwischen einer ersten Filtereigenschaft und einer zweiten Filtereigenschaft hin und her geschaltet werden kann, ohne den Strahlengang zu verlassen. Somit wird sichergestellt, dass in jeder Einstellung der drehbaren Filteranordnung eine Filterung des Lichts nach den Erfordernissen der jeweiligen Untersuchung gewährleistet ist. Da die Filteranordnung selbst im Strahlengang verbleibt, ist es möglich, dies zu implementieren, ohne die Baugröße des Endoskops zu verändern oder alternativ eine Vergrößerung gering zu halten.

Erfindungsgemäß weist die Filteranordnung zwei, drei oder vier plane optische Filter auf, die von der Halterung gehalten werden und um 90° zueinander gedreht angeordnet sind, wobei in der ersten Solldrehposition wenigstens eines der planen optischen Filter mit der ersten Filtereigenschaft und in der zweiten Solldrehposition wenigstens ein anderes der planen optischen Filter mit der zweiten Filtereigenschaft im Strahlengang steht. Die Minimalanzahl von optischen Filtern in diesem Fall sind zwei optische Filter, die um 90° zueinander gedreht an den Seiten eines Vierecks angeordnet sind, so dass bei Drehung der Filteranordnung jeweils wenigstens eines der beiden optischen Filter im Strahlengang eingeführt ist und das jeweils andere parallel zum Strahlengang außerhalb des Strahlengangs positioniert ist. Aufgrund der geringen optischen Dicke der planen optischen Filter entfällt außerdem die Notwendigkeit der Nachfokussierung bei Wechsel der Filtereigenschaft bzw. Drehung der Filteranordnung.

Erfindungsgemäß ist die Drehachse der Filteranordnung senkrecht zu dem wenigstens einen Strahlengang des Endoskops ausgerichtet.

Die vorgenannte Ausführungsform ist sowohl bei Endoskopen mit einem Strahlengang als auch bei Stereo-Endoskopen einsetzbar, welche ggf. abschnittsweise, zwei getrennte optische Strahlengänge aufweisen. So kann die Filteranordnung auch an einer Stelle im Endoskop angeordnet sein, an der eine Aufspaltung in zwei parallele Strahlengänge noch nicht stattgefunden hat. Gegebenenfalls kann pro Strahlengang auch jeweils eine Filteranordnung eingesetzt werden.

In einer Ausführungsform, die nicht unter den Gegenstand der Ansprüche fällt, ist das Endoskop als Stereo-Endoskop mit zwei parallelen Strahlengängen ausgebildet und die Filteranordnung als Filterrad mit zwei Paaren von Filtern ausgebildet, wobei die Filter eines ersten Filterpaares die erste Filtereigenschaft aufweisen und die Filter eines zweiten Filterpaares die zweite Filtereigenschaft aufweisen, wobei die Drehachse der Filteranordnung einer Mittelachse zwischen den Strahlengängen entspricht und/oder parallel zur Mittelachse liegt, wobei die Filter des ersten Filterpaares in der ersten Solldrehposition mit den Strahlengängen fluchten und die Filter des zweiten Filterpaares in der zweiten Solldrehposition mit den Strahlengängen fluchten. Im Unterschied zum Stand der Technik ist hier das Filterrad mit seinen zwei Paaren von optischen Filtern in den Strahlengängen des Stereo-Endoskops angeordnet, wo genügend Platz für dieses Filterrad vorhanden ist. Eine Filterung des Illuminationslichtes ist nicht notwendig, so dass der Verlust von Illuminationsintensität begrenzt werden kann.

In Ausführungsformen umfasst das Endoskop eine Stellvorrichtung, mittels der die Filteranordnung drehbar ist. Die Stellvorrichtung befindet sich üblicherweise am Handgriff oder in einer Steuervorrichtung für das endoskopische System und ermöglicht das Umschalten zwischen den Solldrehpositionen und somit von der ersten Filtereigenschaft zur zweiten Filtereigenschaft und zurück.

Die Stellvorrichtung umfasst in Ausführungsformen einen als äußeren Magnetring ausgebildeten Stellring, welcher als Teil einer Magnetkupplung mit einem inneren Magnetring wirkverbunden ist, welcher, insbesondere über eine Mechanik, insbesondere eine Zahnradmechanik, mit der Halterung der Filteranordnung wirkverbunden ist. Rotatorische Magnetkupplungen sind bei Endoskopen bekannt. Sie weisen einen äußeren, mit der Hand fassbaren Stellring, der als Magnetring ausgebildet ist, und einen inneren Magnetring im hermetischen Teil des Endoskops auf, die über die Anordnung von umfänglich verteilten Magnetpolschuhen oder Einzelmagneten eine Magnetfeldgeometrie herstellen, die in Umfangsrichtung variiert. Diese Inhomogenität sorgt dafür, dass eine Drehung des äußeren Magnetrings zu einer gleich großen Drehung des inneren Magnetrings führt, welche mechanisch auf die Halterung der optischen Filteranordnung übertragen werden kann. Gegebenenfalls, im Fall eines Filterrads, kann der innere Magnetring auch Teil des Filterrads sein.

Alternativ ist vorgesehen, dass die Stellvorrichtung einen elektrischen Motor und eine Steuereinheit für die Motorsteuerung sowie eine Bedieneinheit umfasst.

Um einen sicheren Sitz der jeweiligen Halterung der optischen Filteranordnung in den beiden alternativen Solldrehpositionen zu gewährleisten, ist in Ausführungsformen ein Rastmechanismus umfasst, der ausgebildet ist, die Filteranordnung in der ersten Solldrehposition bzw. in der zweiten Solldrehposition zu fixieren, bis eine Kraft aufgewendet wird, die die Fixierung überwindet.

Schließlich wird die der Erfindung zugrundeliegende Aufgabe auch durch eine Verwendung des Endoskops gemäß der Erfindung gelöst.

Die Verwendung bezieht sich auf das erfindungsgemäße Endoskop und teilt daher dessen Vorteile, Eigenschaften und Merkmale.

Weitere Merkmale der Erfindung werden aus der Beschreibung erfindungsgemäßer Ausführungsformen zusammen mit den Ansprüchen und den beigefügten Zeichnungen ersichtlich.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des durch den Umfang der Ansprüche definierten Schutzbereichs anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Querschnittsdarstellung durch einen Teil eines Endoskops nach dem Stand der Technik,
- Fig. 2a), b): eine schematische Querschnittsdarstellung durch einen Teil eines nicht erfindungsgemäßen Endoskops mit einer drehbaren Filteranordnung sowie eine Prinzipdarstellung der Filteranordnung,
- Fig. 3: eine schematische Darstellung der Drehung einer drehbaren Filteranordnung,
- Fig. 4: eine schematische Querschnittsdarstellung durch einen Teil eines erfindungsgemäßen Endoskops mit einer Filteranordnung,
- Fig. 5a), b): schematische Darstellungen der Filteranordnung gemäß Fig. 4,
- Fig. 6: einen Strahlengang eines seitwärtsblickenden Stereo-Endoskops nach dem Stand der Technik sowie
- Fig. 7: eine schematische Darstellung der Anpassung eines Filterrads für einen Strahlengang gemäß Fig. 6.

In den Zeichnungen sind jeweils gleiche oder gleichartige Elemente und/oder Teile mit denselben Bezugsziffern versehen, so dass von einer erneuten Vorstellung jeweils abgesehen wird.

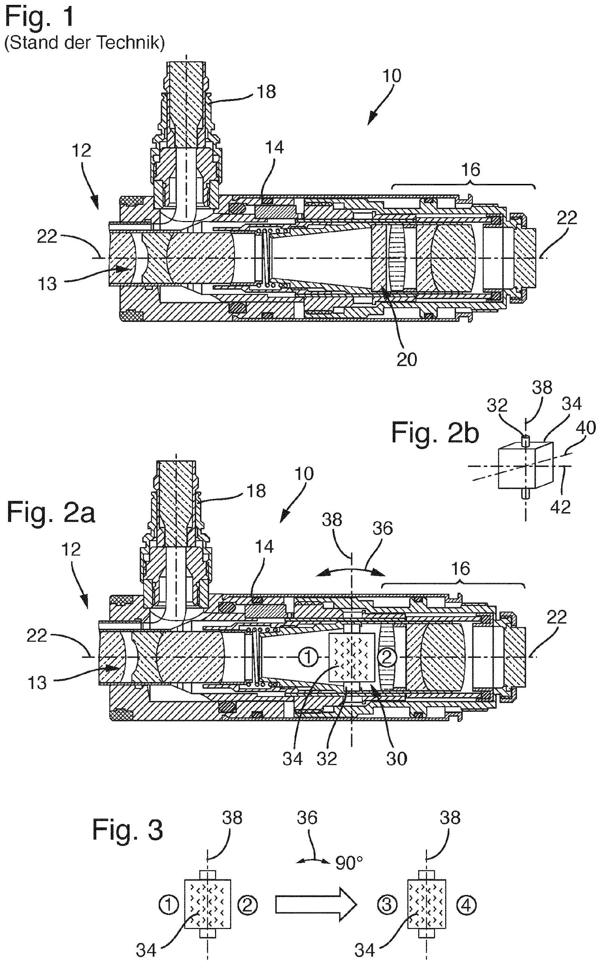

Fig. 1 zeigt schematisch im Querschnitt einen Teil eines Endoskops 10, welches aus dem Stand der Technik bekannt ist. Dabei handelt es sich um ein IR-Laparoskop mit einem (nicht dargestellten) Endoskopschaft mit einem Objektiv 13 bzw. ggf. einer Reihe von Linsenumkehrsätzen, die entlang einer optischen Achse 22 oder Mittelachse des optischen Systems des Endoskops 10 angeordnet sind. Der gezeigte Teil befindet sich im Handgriff des Endoskops 10, an dessen Gehäuse 14 auch eine Beleuchtungseinheit 18 vorhanden ist, welche Licht erzeugt, das in nach distal geleiteten Lichtleitfasern eingeleitet wird (ohne Bezugszeichen). Das Licht, welches von links durch den ansatzweise dargestellten Schaft 12 durch das Endoskop 10 propagiert, gelangt zu einem als IR-Filter ausgebildeten optischen Filter 20, durch welches nur die spektralen Anteile des Lichts im IR-Bereich durchgelassen werden und in die Linsengruppe des Okulars 16 eintreten, die nicht ausgefiltert wurden. Das optische Filter 20 verbleibt im Strahlengang, so dass die Filtereigenschaften in diesem Endoskop 10 festgelegt sind.

Fig. 2a) stellt ein modifiziertes Endoskop, allerdings nicht gemäß der Erfindung, auf der Grundlage des in Fig. 1 gezeigten bekannten Endoskops dar, bei welchem das IR-Filter 20 durch eine nicht erfindungsgemäße optische Filteranordnung 30 ersetzt wurde, die um eine Drehachse 38 drehbar ist. Die optische Filteranordnung 30 umfasst eine Halterung 32, welche die Drehung 36 um die Drehachse 38 erlaubt und ein im Wesentlichen quaderförmiges optisches Filter 34 hält, das in Fig. 2b) prinzipiell schematisch dargestellt ist.

Das im Wesentlichen quaderförmige optische Filter 34 weist zwei Hauptachsen 40, 42 auf, die zur Drehachse 38 und zueinander senkrecht stehen. Diese verlaufen mittig durch die Seitenflächen des im Wesentlichen quaderförmigen optischen Filters 34. Durch Drehung des optischen Filters 34 um die Drehachse 38 um 90° wird abwechselnd die Hauptachse 40 oder die Hauptachse 42 mit der optischen Achse des optischen Systems des Endoskops 10 parallel gebracht, so dass nur die Beschichtungen an den jeweils aktiven Ein- und/oder Austrittsflächen des optischen Filters 34 für die Filterwirkung mit der jeweils eingestellten Filtereigenschaft sorgen. Von den beiden Seitenflächen, durch die die Hauptachse 40 verläuft, ist wenigstens eine so beschichtet, dass sich die erste Filtereigenschaft einstellt, während von den beiden Seitenflächen, durch die die Hauptachse 42 verläuft, wenigstens eine so beschichtet ist, dass sich die zweite Filtereigenschaft einstellt. Die erste Filtereigenschaft kann beispielsweise das Durchlassen von sichtbarem Licht und Blockieren von IR-Licht sein, während die zweite Filtereigenschaft das Durchlässen von Infrarotlicht ist. Dies ist nicht auf eine bestimmte Auswahl oder Kombination von Filtereigenschaften beschränkt, die Filtereigenschaften müssen sich nur unterscheiden.

Fig. 3 zeigt die Wirkung einer Drehung 36 um 90° um die Drehachse 38 des optischen Filters 34. So befinden sich in einem ersten Zustand die Seitenflächen 1 und 2 und im zweiten Zustand die Seitenflächen 3 und 4 im Strahlengang, wobei sich einerseits die Seitenflächen 1 und 2 gegenüberliegen und andererseits sich die Seitenflächen 3 und 4 gegenüberliegen. In dem ersten Zustand bzw. Solldrehposition weist das optische Filter 34 eine größere Dicke auf als im zweiten Zustand bzw. der zweiten Solldrehposition. Die Dicken sind in den beiden Richtungen so gewählt, dass eine Drehung ohne eine Neufokussierung des optischen Systems erfolgen kann, mithin an die Filtereigenschaft angepasst wird.

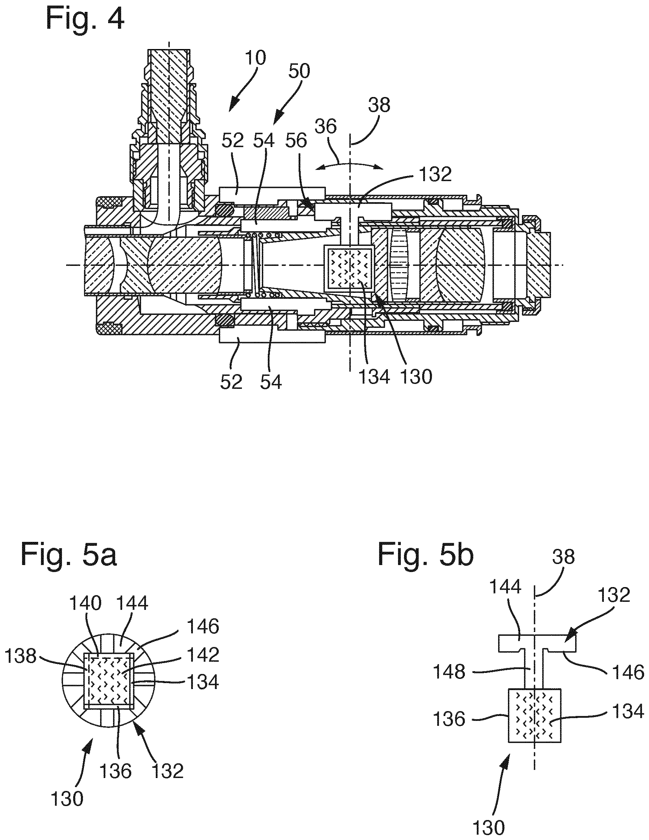

Fig. 4 zeigt ein erfindungsgemäße Ausführungsbeispiel eines Endoskops 10 mit drehbarer Filteranordnung 130. Im Unterschied zu der Filteranordnung 30 gemäß Fig. 2 und 3 sind in der Halterung 132 mehrere planare bzw. plane optische Filter 134, 136, ggf. auch 138, 140, angeordnet, welche jeweils die erste bzw. zweite Filtereigenschaft erzeugen. Diese planaren Filter 134, 136 sind um 90° gedreht zueinander angeordnet und entsprechen den Seitenflächen eines Würfels.

In Fig. 4 ist auch eine Stellvorrichtung 50 mit einem Stellmechanismus dargestellt, welcher für die Ausführungsform der Fig. 2 und 3 ebenfalls anwendbar ist. Es handelt sich um eine Magnetkupplung, welche einen äußeren Magnetring 52 als Stellring aufweist, der mit der Hand eines Operateurs anzufassen ist und um die Längsachse des Endoskops zu drehen ist, sowie einen mit diesem magnetisch wechselwirkenden inneren Magnetring 54, der durch die Drehung des äußeren Magnetrings 52 mitgenommen wird. Der innere Magnetring weist an seinem proximalen Ende ein Zahnradprofil auf, welches im Zusammenhang mit einem Zahnradprofil an der Haltung 132 einen Zahnradmechanismus bzw. eine Zahnradmechanik 56 ausbildet. Eine Drehung des inneren Magnetrings 54 um die optische Achse bzw. zentrale Achse des Endoskops sorgt somit dafür, dass die Halterung 132 mit den an der Halterung angeordneten optischen Filtern um die zur optischen Achse senkrechte Drehachse 38 gedreht wird.

Die Halterung 132 selber ist in Fig. 5 näher dargestellt. Sie umfasst eine Halterungsscheibe 144, welche das Zahnradprofil 146 aufweist, sowie einen zentralen Halterungsschaft 148, der von der Halterungsscheibe 144 ausgeht und an seinem anderen Ende die 2, 3 oder 4 optischen Filter 134, 136, ggf. 138, 140, aufweist. In dem Leerraum zwischen den optischen Filtern 134, 136, 138, 140 ist kein Material vorhanden. Die Tatsache, dass die jeweils zweiten optischen Filter 138, 140 der beiden Paare optional sind, ist in Fig. 5a) dadurch deutlich gemacht, dass diese nur gestrichelt angedeutet sind.

An der Halterungsscheibe 144 können auch umfänglich Nuten in einem Abstand von 90° zueinander angebracht sein, die mit einem Feder-Rastmechanismus (nicht dargestellt) zusammenwirken, um eine sichere Positionierung der Filteranordnung in den Solldrehpositionen zu gewährleisten.

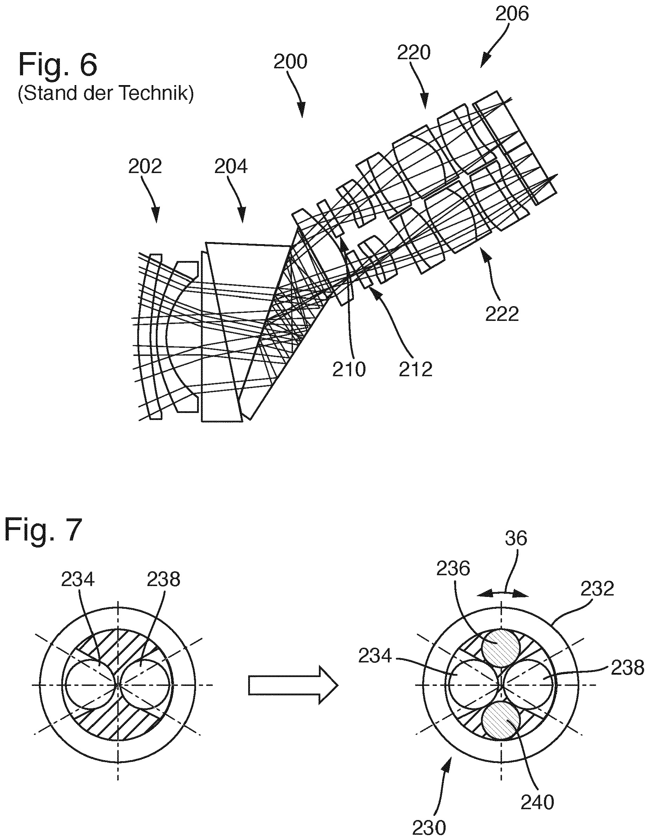

In Fig. 6 ist ein Strahlengang eines seitwärtsblickenden Stereo-Endoskops nach dem Stand der Technik dargestellt. Dieser weist ein Eingangslinsensystem 202 mit einem gebogenen Eingangsfenster und einer Eingangslinse auf, sowie eine Umlenkprismeneinheit 204, an die sich eine weitere Linse sowie zwei parallele Linsensysteme 206 eines ersten Strahlengangs 220 und eines zweiten Strahlengangs 222 anschließen. Zwischen der direkt hinter den Umlenkprismen 204 angeordneten Linse und den parallelen Linsensystemen 206 ist jeweils ein optisches Filter 210, 212, welches beispielsweise als Infrarotfilter ausgebildet sein kann, angeordnet.

In Fig. 7 ist auf der linken Seite die Filteranordnung aus dem optischen System 200 der Fig. 6 in Frontalansicht dargestellt. Die rechts dargestellte nicht erfindungsgemäße Filteranordnung 230 umfasst demgegenüber ein Filterrad 232, welches um die in der Normale der Bildebene verlaufende Drehachse (ohne Bezugszeichen) gedreht werden kann (Bezugszeichen 36), wobei eine Drehung um 90° die beiden Filter 234, 238 aus den Strahlengängen herausrotiert und die Filter 236, 240 in die Strahlengänge hineinrotiert. Eine Änderung der Geometrie ist hierfür nicht notwendig. Das Filterrad 232 kann mit dem inneren Magnetring 54 aus dem Ausführungsbeispiel der Fig. 4 mechanisch verbunden sein oder dieses umfassen. Das Filterrad 232 kann Nuten oder ähnliche Mittel im Abstand von 90° für einen Rastmechanismus aufweisen.

Im Falle, dass das gezeigte optische System am distalen Ende eines langen Endoskopschafts angeordnet ist, kann auch eine mechanische Verbindung durch den Endoskopschaft für die Drehung des Filterrads 232 vorgesehen sein, oder es kann ein elektrischer Stellmotor vorgesehen sein oder andere geeignete Mittel eingesetzt werden.

### Bezugszeichenliste

- 10: Endoskop
- 12: Schaft
- 13: Objektiv
- 14: Gehäuse
- 16: Okular
- 18: Beleuchtungseinheit
- 20: optisches Filter
- 22: optische Achse
- 30: optische Filteranordnung
- 32: Halterung
- 34: optisches Filter
- 36: Drehung
- 38: Drehachse
- 40, 42: Hauptachse
- 50: Stellvorrichtung
- 52: Stellring
- 54: innerer Magnetring
- 56: Zahnradmechanik
- 130: optische Filteranordnung
- 132: Halterung
- 134, 138: optisches Filter
- 136, 140: optisches Filter
- 142: Leerraum
- 144: Halterungsscheibe
- 146: Zahnradprofil
- 148: Halterungsschaft
- 200: optisches System eines Stereo-Endoskops
- 202: Eingangslinsensystem
- 204: Umlenkprismen
- 206: parallele Linsensysteme
- 210, 212: optisches Filter
- 220: erster Strahlengang
- 222: zweiter Strahlengang
- 230: optische Filteranordnung
- 232: Filterrad
- 234, 238: optisches Filter
- 236, 240: optisches Filter

## Patentansprüche

1. Endoskop (10) mit einer optischen Filteranordnung (130), welche in einer Halterung (132) mehrere optische Filter (134, 136, 138, 140) mit unterschiedlichen Filtereigenschaften umfasst, und in wenigstens einem Strahlengang des Endoskops (10) in Lichteinfallsrichtung in oder hinter einem Objektiv (13) des Endoskops (10) und vor einer Bildaufnahmeeinheit des Endoskops (10) oder einem Okular (16) des Endoskops (10) um eine Drehachse (38) drehbar angeordnet ist, wobei die Filteranordnung (130) ausgebildet ist, bei Drehung der Filteranordnung (130) um die Drehachse (38) von einer ersten Solldrehposition in eine zweite Solldrehposition einen Wechsel von einer ersten Filtereigenschaft zu einer zweiten Filtereigenschaft zu vollziehen, wobei die Filteranordnung (130) zwei, drei oder vier plane optische Filter (134, 136, 138, 140) aufweist, die von der Halterung (130) gehalten werden und zueinander gedreht angeordnet sind, wobei in der ersten Solldrehposition wenigstens eines der planen optischen Filter (134, 138) mit der ersten Filtereigenschaft und in der zweiten Solldrehposition wenigstens ein anderes der planen optischen Filter (136, 140) mit der zweiten Filtereigenschaft im Strahlengang steht, wobei die Drehachse (38) der Filteranordnung (30, 130) senkrecht zu dem wenigstens einen Strahlengang des Endoskops (10) ausgerichtet ist,
**dadurch gekennzeichnet, dass**
die Drehung der Filteranordnung (130) eine Drehung der Filteranordnung (130) um 90° ist und die zwei, drei, oder vier planen optischen Filter (134, 136, 138, 140) um 90° zueinander gedreht angeordnet sind.

2. Endoskop (10) nach Anspruch 1, **dadurch gekennzeichnet, dass** das Endoskop (10) eine Stellvorrichtung (50) umfasst, mittels der die Filteranordnung (130) drehbar ist.

3. Endoskop (10) nach Anspruch 2, **dadurch gekennzeichnet, dass** die Stellvorrichtung (50) einen als äußerer Magnetring ausgebildeten Stellring (52) umfasst, welcher als Teil einer Magnetkupplung des Endoskops (10) mit einem inneren Magnetring (54) wirkverbunden ist, welcher, insbesondere über eine Mechanik, insbesondere eine Zahnradmechanik (56), mit der Halterung (132) der Filteranordnung (30, 130, 230) wirkverbunden ist.

4. Endoskop (10) nach Anspruch 2, **dadurch gekennzeichnet, dass** die Stellvorrichtung einen elektrischen Motor und eine Steuereinheit für die Motorsteuerung sowie eine Bedieneinheit umfasst.

5. Endoskop (10) nach einem der Ansprüche 1 bis 4, **dadurch gekennzeichnet, dass** ein Rastmechanismus umfasst ist, der ausgebildet ist, die Filteranordnung (130) in der ersten Solldrehposition bzw. in der zweiten Solldrehposition zu fixieren, bis eine Kraft aufgewendet wird, die die Fixierung überwindet.

6. Verwendung eines Endoskops (10) nach einem der Ansprüche 1 bis 5.

## Claims

1. An endoscope (10) with an optical filter arrangement (130), which comprises in a holder (132) multiple optical filters (134, 136, 138, 140) with different filter properties, and is arranged rotatably about an axis of rotation (38) in at least one beam path of the endoscope (10) in the direction of light incidence in or behind an objective (13) of the endoscope (10) and in front of an image capturing unit of the endoscope (10) or an eyepiece (16) of the endoscope (10), wherein the filter arrangement (130) is designed to perform a change from a first filter property to a second filter property when the filter arrangement (130) is rotated about the axis of rotation (38) from a first target rotational position to a second target rotational position, wherein the filter arrangement (130) has two, three or four flat optical filters (134, 136, 138, 140) which are held by the holder (130) and are arranged rotated in relation to each other, wherein in the first target rotational position at least one of the flat optical filters (134, 138) with the first filter property and in the second target rotational position at least one other of the flat optical filters (136, 140) with the second filter property lies in the beam path, wherein the axis of rotation (38) of the filter arrangement (30, 130) is oriented perpendicular to the at least one beam path of the endoscope (10), **characterized in that** the rotation of the filter arrangement (130) is a rotation of the filter arrangement (130) by 90° and the two, three or four flat optical filters (134, 136, 138, 140) are arranged rotated by 90° in relation to each other.

2. The endoscope (10) according to claim 1, **characterized in that** the endoscope (10) comprises an adjusting device (50) by means of which the filter arrangement (130) can be rotated.

3. The endoscope (10) according to claim 2, **characterized in that** the adjusting device (50) comprises an adjusting ring (52) designed as an outer magnetic ring which is operatively connected to an inner magnetic ring (54) as part of a magnetic coupling, which inner magnetic ring is operatively connected to the holder (132) of the filter arrangement (30, 130, 230), in particular via a mechanical unit, in particular a gearwheel mechanical unit (56).

4. The endoscope (10) according to claim 2, **characterized in that** the adjusting device comprises an electric motor and a control unit for the motor control as well as an operating unit.

5. The endoscope (10) according to one of claims 1 to 4, **characterized in that** a latching mechanism is comprised which is designed to fix the filter arrangement (130) in the first target rotational position or in the second target rotational position until a force is applied that overcomes the fixing.

6. A use of an optical filter arrangement (130) in an endoscope (10) according to one of claims 1 to 5, which comprises a holder (132) which holds multiple optical filters (134, 136, 138, 140) with different filter properties, wherein the filter arrangement (130) is arranged rotatably about an axis of rotation (38) in at least one beam path of the endoscope (10) in the direction of light incidence in or behind an objective (13) of the endoscope (10) and in front of an image capturing unit of the endoscope (10) or an eyepiece (16) of the endoscope (10), wherein the filter arrangement (130) is designed to perform a change from a first filter property to a second filter property when the filter arrangement (30, 130, 230) is rotated by 90° about the axis of rotation (38) from a first target rotational position to a second target rotational position.

## Revendications

1. Endoscope (10) avec un agencement de filtres optiques (130), qui comprend dans un support (132) plusieurs filtres optiques (134, 136, 138, 140) avec différentes propriétés de filtrage, et qui est disposé de manière à pouvoir tourner autour d'un axe de rotation (38) dans au moins un trajet optique de l'endoscope (10) dans la direction d'incidence de la lumière dans ou derrière un objectif (13) de l'endoscope (10) et devant une unité de prise de vue de l'endoscope (10) ou un oculaire (16) de l'endoscope (10), l'agencement de filtres (130) étant conçu de façon à passer d'une première propriété de filtre à une deuxième propriété de filtre lors de la rotation de l'agencement de filtres (130) autour de l'axe de rotation (38) d'une première position nominale de rotation à une deuxième position nominale de rotation, l'agencement de filtres (130) comprenant deux, trois ou quatre filtres optiques plans (134, 136, 138, 140) qui sont maintenus par le support (130) et sont disposés de façon rotative les uns par rapport aux autres, au moins l'un des filtres optiques plans (134, 138) qui présente la première propriété de filtrage dans la première position nominale de rotation et au moins un autre des filtres optiques plans (136, 140) qui présente la deuxième propriété de filtrage dans la deuxième position nominale de rotation se trouvant dans le trajet optique, l'axe de rotation (38) de l'agencement de filtres (30, 130) étant orienté perpendiculairement audit au moins un trajet optique de l'endoscope (10), **caractérisé en ce que**
la rotation de l'agencement de filtres (130) est une rotation de l'agencement de filtres (130) sur 90° et les deux, trois, ou quatre filtres optiques plans (134, 136, 138, 140) sont agencés en étant tournés de 90° les uns par rapport aux autres.

2. Endoscope (10) selon la revendication 1, **caractérisé en ce que** l'endoscope (10) comprend un dispositif de réglage (50) au moyen duquel l'agencement de filtres (130) peut être tourné.

3. Endoscope (10) selon la revendication 2, **caractérisé en ce que** le dispositif de réglage (50) comprend une bague de réglage (52) conçue sous la forme d'une bague magnétique extérieure qui, en tant que partie d'un couplage magnétique de l'endoscope (10), est en liaison active avec une bague magnétique intérieure (54), laquelle est en liaison active avec le support (132) de l'agencement de filtres (30, 130, 230), en particulier par l'intermédiaire d'un mécanisme, en particulier un mécanisme à roue dentée (56).

4. Endoscope (10) selon la revendication 2, **caractérisé en ce que** le dispositif de réglage comprend un moteur électrique et une unité de commande pour la commande du moteur ainsi qu'une unité de contrôle.

5. Endoscope (10) selon l'une des revendications 1 à 4, **caractérisé en ce qu'**il comprend un mécanisme d'encliquetage configuré pour immobiliser l'agencement de filtres (130) dans la première position nominale de rotation ou dans la deuxième position nominale de rotation jusqu'à ce qu'une force soit appliquée pour surmonter l'immobilisation.

6. Utilisation d'un endoscope (10) selon l'une quelconque des revendications 1 à 5.
